# EUROPEAN PATENT SPECIFICATION

(11) **EP 3 061 359 B1**
(45) Date of publication and mention of the grant of the patent: **03.10.2018**
(21) Application number: 16158159.0
(22) Date of filing: 15.05.2007
(51) Int. Cl.: A24F 47/00

(54) **AEROSOL ELECTRONIC CIGARETTE**
ELEKTRONISCHE AEROSOL-ZIGARETTE
CIGARETTE ÉLECTRONIQUE À AÉROSOL

(30) Priority: 16.05.2006 CN 200620090805 U
(43) Date of publication of application: 31.08.2016
(62) Divisional of application: 14155503.7
(73) Proprietor: Fontem Holdings 1 B.V., 1083 HN Amsterdam (NL)
(72) Inventor: HAN, Li, Hong Kong (CN)
(74) Representative: Gulde & Partner

(56) References cited:
- EP-A1- 0 845 220
- WO-A1-2005/099494
- US-A- 4 922 901

## Description

### Technical Field

The present invention relates to an atomizer assembly for an aerosol electronic cigarette.

### Background Art

Today when "smoking is harmful to your health" has become a common sense, there are one billion people smoking cigarettes, and this figure is still rising. On March 1, 2003, the World Health Organization (WHO) issued the first international smoking ban - Framework Convention on Tobacco Control. According to WHO's data, smoking causes 4,900,000 deaths each year. Smoking causes serious respiratory system diseases and cancers, though it is a hard job to persuade the smokers to completely quit smoking.

Nicotine is a micro-molecular alkaloid, which is basically harmless to human bodies in small dosages. Plus, its half-life period is extremely short in blood. Tar is the major harmful substance in tobacco. Tobacco tar comprises of several thousands of ingredients, dozens of which are carcinogenic substances. It has now been proved that second hand smoking is even more harmful to those who don't smoke.

Inventors have used relatively pure nicotine to create cigarette substitutes that contain nicotine but do not contain harmful tar, such as "Cigarette Patches", "Nicotine Gargle", "Nicotine Aerosol in a High Pressure Tank with Propellant", "Nicotine Chewing Gum", and "Nicotine Beverages". These products are not as harmful as they lack tar, but the nicotine in such products is absorbed very slowly. As a result, a peak concentration can't be effectively established in the blood, and smokers can't be satisfied to the full. In addition, smokers are deprived of the "smoking" habit. Therefore, these products are not real cigarette substitutes for helping smokers to quit smoking.

Electronic cigarettes may resolve the above-mentioned issue. However, existing electronic cigarettes don't provide ideal aerosol effects, and their atomizing efficiency is not high.

EP 0845220 is an example of a proposed design for a flavor generation article for simulating smoking. In EP 0845220 liquid for inhalation is sprayed from a liquid discharge port onto a porous liquid absorbing layer provided on the surface of a ceramic heater. The porous liquid absorbing layer retains the liquid against the surface enabling the liquid to be heated and gasified by the heater.

US4922901 discloses a drug delivery article in which a resistance heating element having a high surface area and an absorbent, porous, wettable character is impregnated with a liquid aerosol forming substance. Suitable heating elements preferably have surface areas about 50m²/g or higher. Preferred resistance heating elements include carbon filament yarns, carbon felts and activated carbon felts.

WO2005/0099494 discloses an atomizer for an electronic cigarette comprising a porous body soaked in nicotine liquid mounted on a piezoelectric element. Air passes into an atomization cavity inside the porous body via an ejection hole causing droplets of liquid to be drawn from the porous body and entrained in the high speed airflow. The droplets are then subjected to ultrasonic vibration and further atomized by a heating element present within the atomizing cavity. In some embodiments either the heating element or the piezoelectric element may be omitted.

### Contents of invention

To overcome the above-mentioned disadvantages, the present invention has been designed to provide an atomizer assembly for an aerosol electronic cigarette that substitutes for cigarettes and helps smokers to quit smoking.

In accordance with the present invention, an atomizer assembly for an aerosol electronic cigarette is provided comprising: a porous component; and a heating body in the form of a heating wire; the atomizer assembly includes a support member having a run-through hole; the porous component is mounted on the support member and is wound with the heating wire in a part that is on the side in the axial direction of the run-through hole.

In embodiments of the present invention the porous component may be made of foamed nickel, stainless steel fiber felt, macromolecular polymer foam or foamed ceramics.

In embodiments of the present invention the heating wire may be made of platinum wire, nickel-chromium alloy wire or iron-chromium alloy wire containing rare earth, or may be flaked.

### Description of Drawings

Figure 1 is a side section view of an electronic cigarette.
Figure 2 is a view of the electronic cigarette of Figure 1 with the cigarette bottle assembly of the electronic cigarette separated from a shell containing an atomizer assembly and a battery assembly.
Figure 3 is a diagram of the axial structure of the cigarette bottle assembly, illustrating a ventilating groove on a peripheral surface of the cigarette holder shell of the cigarette bottle assembly.
Figure 4 is a side section view of the cigarette bottle assembly, illustrating the structure of an air channel.
Figure 5 is a diagram of the axial structure of an atomizer in accordance with an embodiment of the present invention.
Figure 6 is a side section view of the atomizer of Figure 5.

### Specific Mode for Carrying Out the Invention

This invention is further described as follows on the basis of the drawings.

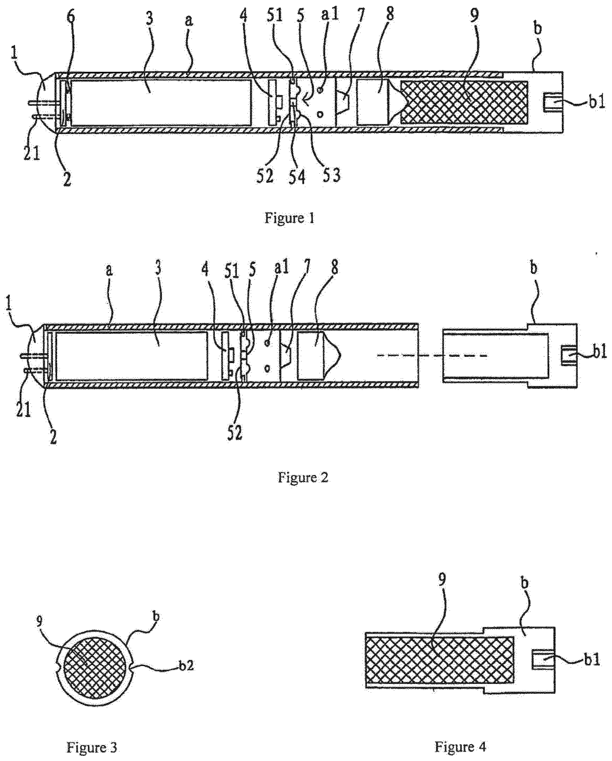

Figures 1 and 2 show an aerosol electronic cigarette, which includes a battery assembly, an atomizer assembly and a cigarette bottle assembly, and also includes a shell (a), which is hollow and integrally formed. The battery assembly connects with the atomizer assembly and both are located in the shell (a). A cigarette bottle assembly is detachably mounted in one end of the shell (a). The cigarette bottle assembly fits with the atomizer assembly. The shell (a) has through-air-inlets (a1).

The battery assembly includes a battery (3), an operating indicator (1), an electronic circuit board (4), and airflow sensor (5), which are connected with the battery (3). It also includes a check valve (7). The signal output of the airflow sensor (5) is connected with the electronic circuit board (4). The battery (3) is a rechargeable battery, which may be either a rechargeable polymer lithium ion battery or a rechargeable lithium ion battery. The airflow sensor (5) may be a semiconductor force-sensitive chip capacitance sensor or an inductance sensor. The rechargeable battery (3) has a flexibly connected charging plug (2). The blades (21) of the charging plug (2) come out of the other end of the shell (a). Between the charging plug (2) and rechargeable battery (3) is a spring (6), which lies against the body of the rechargeable battery (3) on one end, while its free end lies against the charging plug (2), forming a flexible structure, which buffers the charging plug (2) when plugged for charging, thus protecting the rechargeable battery (3) against any damage. Of course, the rechargeable battery (3) in this embodiment has a charging slot on it, which replaces the structure of charging plug (2) to perform the charging function and protect the rechargeable battery (3) against any damage. The operating indicator (1) is a LED. In this embodiment, there are two LEDs. The electronic circuit board (4) includes an electronic switch circuit, which controls the electric circuit according to the input signals.

As shown in Figure 1 and 2 , the airflow sensor (5) has a silica gel corrugated membrane (53), which connects with magnetic steel (54) with a reed relay (52) on one of its ends. Both ends of the said reed relay (52) correspond to the relay electrodes (51) respectively.

In this embodiment, the battery assembly and atomizer assembly are mutually connected and then installed inside the integrally formed shell (a) to form a one-piece part. The rechargeable battery (3) may be charged without frequent change of battery. The user just needs to plug the cigarette bottle assembly into the open end of the shell (a), for easy use and very easy change.

As shown in Figures 3 and 4, the cigarette bottle assembly includes a hollow cigarette holder shell (b), and a porous component for liquid storage (9) inside the shell (b). The porous component for liquid storage (9) is made of materials suitable for liquid storage such as polypropylene fiber, terylene fiber or nylon fiber. Alternatively, it may be a plastic foam molding or a column of multi-layer plates made through plastic injection with polyvinyl chloride, polypropylene and polycarbonate. One end of the cigarette holder shell (b) plugs into the shell (a), and the outer peripheral surface of the cigarette holder shell (b) has an inward ventilating groove (b2). On one end surface of the cigarette holder shell (b), there is an air channel (b1) extending inward. An air channel (b1) is located in the center on the surface of one end of shell (b).

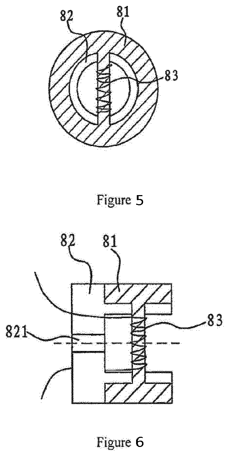

As shown in Figures 5 and 6, the atomizer assembly (8), includes a support member (82). A porous component (81) set on the support member (82), and a heating wire (83) is wound on the porous component (81). The support member (82) has a run-through hole (821) on it. The porous component (81) is wound with the heating wire (83) in the part that is on the side in the axial direction of the run-through hole (821). One end of the porous component (81) fits with the cigarette bottle assembly. The porous component (81) is made of foamed nickel, stainless steel fiber felt, macromolecular polymer foam or foamed ceramics.

As shown in Figure 1, one end of the porous component (81) lies against one end surface of the said porous component for liquid storage (9), and contacts the porous component for liquid storage (9). The porous component for liquid storage (9) of the cigarette bottle assembly and the porous component (81) of the atomizer (8) contact each other to achieve the capillary impregnation for liquid supply. It absorbs the cigarette liquid from the porous component for liquid storage (9).

When the smoker smokes, the cavity of the cigarette holder shell (b) is in the negative pressure state. In the shell (b), one end of the airflow sensor (5) forms a normal pressure cavity, while the other end forms a negative pressure cavity. The air pressure difference between the normal pressure cavity and negative pressure cavity or the high-speed airflow enables the magnetic steel (54) of the airflow sensor (5) to drive the reed relay (52) to contact the relay electrode (51). The electric circuit is electrified, and the electronic switch circuit on the electronic circuit board (4) is electrified.

## Claims

1. An atomizer assembly (8) for an aerosol electronic cigarette, comprising:
a porous component (81); and
a heating body in the form of a heating wire (83);
wherein the atomizer assembly (8) includes a support member (82) having a run-through hole (821);
**characterized in that**: the porous component (81) is mounted on the support member (82) and is wound with the heating wire (83) in a part that is on the side in the axial direction of the run-through hole (821).

2. An atomizer assembly (8) in accordance with claim 1 wherein the porous component (81) is made of foamed nickel, stainless steel fiber felt, macromolecular polymer foam or foamed ceramics.

3. An atomizer assembly (8) in accordance with claim 1 or claim 2 wherein the heating wire (83) is made of platinum wire, nickel-chromium alloy wire or iron-chromium alloy wire containing rare earth elements or is flaked.

## Patentansprüche

1. Zerstäuberanordnung (8) für eine elektronische Aerosol-Zigarette, umfassend:
eine poröse Komponente (81); und
einen Heizkörper in Form eines Heizdrahtes (83);
wobei die Zerstäuberanordnung (8) ein Trägerelement (82) mit einem Durchgangsloch (821) umfasst;
**dadurch gekennzeichnet, dass** die poröse Komponente (81) auf dem Trägerelement (82) angebracht ist und mit dem Heizdraht (83) an einem Teil umwickelt ist, der sich in axialer Richtung auf der Seite des Durchgangslochs (821) befindet.

2. Zerstäuberanordnung (8) nach Anspruch 1, wobei die poröse Komponente (81) aus geschäumtem Nickel, Filz aus rostfreien Edelstahlfaserfilz, makromolekularem Polymerschaumstoff oder geschäumter Keramik hergestellt ist.

3. Zerstäuberanordnung (8) nach Anspruch 1 oder Anspruch 2, wobei der Heizdraht (83) aus Platindraht, Draht aus einer Nickel-Chrom-Legierung oder Draht aus einer Eisen-Chrom-Legierung, der Seltenerdmetalle enthält oder geflockt ist, hergestellt ist.

## Revendications

1. Ensemble atomiseur (8) pour une cigarette électronique à aérosol, comprenant :
un composant poreux (81) ; et
un corps chauffant sous la forme d'un fil chauffant (83),
l'ensemble atomiseur (8) comprenant un élément de support (82) doté d'un trou traversant (821) ;
**caractérisé en ce que** :
le composant poreux (81) est monté sur l'élément de support (82) et ledit composant poreux est enroulé avec le fil chauffant (83) dans une partie, laquelle se trouve sur le côté dans la direction axiale du trou traversant (821).

2. Ensemble atomiseur (8) selon la revendication 1, le composant poreux (81) étant constitué de mousse de nickel, de feutre de fibres d'acier inoxydable, de mousse de polymère macromoléculaire ou de mousse céramique.

3. Ensemble atomiseur (8) selon la revendication 1 ou la revendication 2, le fil chauffant (83) étant constitué de fil de platine, de fil d'alliage nickel-chrome ou de fil d'alliage fer-chrome contenant des éléments de terres rares ou est en paillettes.
